# EUROPEAN PATENT APPLICATION

(11) **EP 4 316 509 A1**
(43) Date of publication of application: **07.02.2024**
(21) Application number: 22775776.2
(22) Date of filing: 24.03.2022
(51) Int. Cl.: A61K 38/54, A61K 9/19, A61K 31/198, A61K 47/26, A61K 47/36, A61P 27/04

(54) **LACHRYMATORY FACTOR GENERATING KIT**

(30) Priority: 25.03.2021 JP 2021051943
(71) Applicant: House Foods Group Inc., Higashiosaka-shi, Osaka 577-8520 (JP)
(72) Inventor: UEYAMA Masae, Higashiosaka-shi, Osaka 577-8520 (JP); AOYAGI Morihiro, Higashiosaka-shi, Osaka 577-8520 (JP); IMAI Shinsuke, Higashiosaka-shi, Osaka 577-8520 (JP)
(74) Representative: Kordel, Mattias
(86) International application number: PCT/JP2022/013962
(87) International publication number: WO 2022/202995

(57) **Abstract**

The present specification discloses a kit and a method that can mildly generate a lachrymatory factor. The present disclosure relates to a lachrymatory factor generating kit 1 comprising (1) a dry PRENCSO composition 2 comprising PRENCSO and a porous carrier carrying it and being dried, and (2) a dry enzyme composition 3 comprising alliinase, a lachrymatory factor synthase (LFS) and a porous carrier carrying them and being dried. The present disclosure also relates to a method for generating a lachrymatory factor, the method comprising contacting water with the dry PRENCSO composition 2 and the dry enzyme composition 3.

## Description

### Technical Field

The present invention relates to a lachrymatory factor generating kit.

The present invention also relates to a method for generating a lachrymatory factor.

### Background Art

Thiopropanal S-oxide, a lachrymatory factor (LF) of onion, is generated by action of an enzyme alliinase on a precursor substance, PRENCSO (S-1-propenyl-cysteine sulfoxide), to generate sulfenic acid (E-1-propensulfenic acid), and isomerization of the sulfenic acid by a lachrymatory factor synthase (LFS).

The lachrymatory factor is known to be industrially useful compounds. For example, the lachrymatory factor is also known to be capable of being utilized as a lachrymal secretion test reagent and for a lachrymal secretion test method, in other words, a dry eye test (Patent Literature 1).

The lachrymatory factor is also known to be capable of being utilized in antimicrobial agents (Patent Literature 2).

The lachrymatory factor has low stability and are easily decomposed, and thus, when a lachrymatory factor is to be industrially utilized, the lachrymatory factor is preferably generated on the site where the lachrymatory factor is utilized.

Patent Literature 3 describes a lachrymatory factor generating kit. The lachrymatory factor generating kit described in Patent Literature 3 comprises a dry enzyme mixture, which is produced by allowing water-absorbing paper to absorb a solution containing alliinase and LFS and drying the resultant, and PRENCSO. Patent Literature 3 describes that PRENCSO is comprised in the kit as an acidic solution so as to be stably stored, and that a lachrymatory factor is generated by dropping the acidic solution of PRENCSO to the dry enzyme mixture, as specific embodiments.

### Citation List

### Patent Literature

Patent Literature 1: WO2005/067907
Patent Literature 2: JP2007-267639A
Patent Literature 3: JP2008-285476A

### Summary of Invention

### Technical Problem

In the method described in Patent Literature 3, which involves generating a lachrymatory factor by application of the PRENCSO acidic solution to the dry enzyme mixture, it was required to measure a determined amount of the PRENCSO acidic solution and then to apply this acidic solution to the dry enzyme mixture.

The present inventors have found the following new problem: the method for generating a lachrymatory factor, by dropping the acidic solution of PRENCSO to the dry enzyme mixture, described in Patent Literature 3, causes a high reaction rate and the lachrymatory factor is generated rapidly, and thus a subject feels pain in an application in the case of using a generated lachrymatory factor to expose to the eye of the subject for collecting tears.

An object of the present invention is to solve these problems.

### Solution to Problem

The present specification discloses the following specific embodiments as solutions to solve the above problems.
[1] A lachrymatory factor generating kit comprising:
   (1) a dry PRENCSO composition comprising PRENCSO and a porous carrier carrying it and being dried; and
   (2) a dry enzyme composition comprising alliinase, a lachrymatory factor synthase (LFS) and a porous carrier carrying them and being dried.
[2] The lachrymatory factor generating kit according to [1], wherein (1) and (2) are integrated.
[3] The lachrymatory factor generating kit according to [1] or [2], wherein (1) further comprises sugar.
[4] The lachrymatory factor generating kit according to [3], comprising 5 parts by mass or more of sugar relative to 1 part by mass of PRENCSO.
[5] A method for generating a lachrymatory factor, the method comprising contacting water with
   (1) a dry PRENCSO composition comprising PRENCSO and a porous carrier carrying it and being dried, and
   (2) a dry enzyme composition comprising alliinase, a lachrymatory factor synthase (LFS) and a porous carrier carrying them and being dried.
[6] The method according to [5], wherein (1) and (2) are integrated.
[7] The method according to [5] or [6], wherein (1) further comprises sugar.
[8] The method according to [7], wherein 5 parts by mass or more of sugar is contained relative to 1 part by mass of PRENCSO.
[9] The method according to any of [5] to [8], wherein the water is an aqueous solution containing a surfactant.

The present specification encompasses the disclosure of Japanese Patent Application No. 2021-051943 serving as the basis for the priority of the present application.

### Advantageous Effects of Invention

The lachrymatory factor generating kit and the method can allow for mild generation of a lachrymatory factor by contacting water. Thus, an ocular pain felt by a subject is suppressed in the case of use in an application where a generated lachrymatory factor is exposed to the eye of the subject for collecting tears.

### Brief Description of Drawing

[Figure 1] Figure 1 illustrates the results of a comparison test of the amount of generated LF (lachrymatory factor), in Experiment 1.
[Figure 2] Figure 2 illustrates the results of a surfactant addition test in Experiment 1.
[Figure 3] Figure 3 illustrates the results of a pain comparison test in Experiment 1.
[Figure 4] Figure 4 illustrates the average value of the remaining ratio of the amount of generated LF (%), with respect to each of an integrated lachrymatory factor generating kit 1 (comprising a PRENCSO pad without lactose) and an integrated lachrymatory factor generating kit 2 (comprising a PRENCSO pad containing lactose), after storage at 4°C for 1 month, 3 months, 6 months, and 12 months in Experiment 2.
[Figure 5] Figure 5 illustrates the average value of the remaining ratio of the amount of generated LF (%), with respect to each of the integrated lachrymatory factor generating kits 1 and 2, after storage at 20°C for 1 month, 3 months, and 6 months in Experiment 2.
[Figure 6] Figure 6 illustrates the average value of the remaining ratio of the amount of generated LF (%), with respect to each of the integrated lachrymatory factor generating kits 1 and 2, after storage at 30°C for 1 month, 3 months, 6 months, and 12 months in Experiment 2.
[Figure 7] Figure 7 is a plan view of a lachrymatory factor generating kit in which a sheet-shaped dry PRENCSO composition and a sheet-shaped dry enzyme composition are stacked and integrated.
[Figure 8] Figure 8 is a cross-sectional view along with a line A-A in Figure 7, of the lachrymatory factor generating kit illustrated in Figure 7.
[Figure 9] Figure 9 is a plan view of a lachrymatory factor generating kit in which a sheet-shaped dry PRENCSO composition and a sheet-shaped dry enzyme composition are adjacently located.
[Figure 10] Figure 10 is a cross-sectional view along with a line B-B in Figure 9, of the lachrymatory factor generating kit illustrated in Figure 9.
[Figure 11] Figure 11 is a plan view of a lachrymatory factor generating kit comprising one carrier, in which a porous carrier carrying PRENCSO and a porous carrier carrying alliinase and LFS are integrated, comprising the dry PRENCSO composition carrying PRENCSO on one portion of the one carrier, and the dry enzyme composition carrying alliinase and LFS on another portion of the one carrier.
[Figure 12] Figure 12 is a cross-sectional view along with a line C-C in Figure 11, of the lachrymatory factor generating kit illustrated in Figure 11.

### Description of Embodiments

One or more embodiments of the invention disclosed herein relate to a lachrymatory factor generating kit comprising
(1) a dry PRENCSO composition comprising PRENCSO and a porous carrier carrying it and being dried, and
(2) a dry enzyme composition comprising alliinase, a lachrymatory factor synthase (LFS) and a porous carrier carrying them and being dried.

Other one or more embodiments of the invention disclosed herein relate to
a method for generating a lachrymatory factor comprising
contacting water with
   (1) a dry PRENCSO composition comprising PRENCSO and a porous carrier carrying it and being dried, and
   (2) a dry enzyme composition comprising alliinase, a lachrymatory factor synthase (LFS) and a porous carrier carrying them and being dried.

The kit and the method according to the present disclosure can allow for the mild generation of a lachrymatory factor (LF) (thio propanal S-oxide) by a simple operation of contacting water. These are easily carried out on the site where a lachrymatory factor is utilized, because the amount of water to be added is not required to be critically controlled.

Herein, the "dry" and "dried" are each not limited to absolute dry where no water is contained in the dry PRENCSO composition and the dry enzyme composition at all, and encompass a state where about 0% by mass to 20% by mass of water is contained. In other words, preferably 20% by mass or less, more preferably 10% by mass or less, more preferably 3% by mass or less, more preferably 2% by mass or less of water may be contained relative to the total amount of the above compositions. The water content in the dry PRENCSO composition and the dry enzyme composition can be measured by absolutely drying the compositions and determining the proportion of the mass decreased by absolute drying, relative to the total mass of the compositions before absolute drying.

Preferable embodiments of the dry PRENCSO composition are described.

PRENCSO (S-1-propenyl-cysteine sulfoxide) may be artificially synthesized, or may be extracted from a natural product. PRENCSO purified by an ion-exchange resin or reversedphase chromatography is preferably used.

The porous carrier in the dry PRENCSO composition is preferably a porous carrier being water absorptive, more preferably a porous carrier containing a hydrophilic polymer such as cellulose that is water absorptive, particularly preferably water absorptive paper. The shape of the porous carrier is not particularly limited, and is preferably a sheet shape. The dry PRENCSO composition, which comprising a porous carrier carrying PRENCSO, can be obtained by allowing the porous carrier to absorb a solution containing PRENCSO, and drying the resultant.

The dry PRENCSO composition preferably further comprise an acid such as hydrochloric acid. Patent Literature 3 describes PRENCSO being stabilized in an acidic aqueous solution.

The dry PRENCSO composition preferably further comprise saccharide. The dry PRENCSO composition comprising saccharide is suitable for long-term storage because of being suppressed in decomposition of PRENCSO during storage, compared to the case of no saccharide comprised. A lachrymatory factor generating kit comprising the dry PRENCSO composition comprising saccharide is preferable because a reduction in amount of a generated lachrymatory factor after long-term storage is suppressed, compared to the case of no saccharide comprised.

The type of saccharide is not particularly limited, and, for example, one or more selected from monosaccharide, disaccharide, polysaccharide and sugar alcohol may be used. Each of monosaccharide, disaccharide, polysaccharide and sugar alcohol encompasses an ester thereof with fatty acid.

Examples of monosaccharide may include one or more selected from glucose and fructose. Examples of disaccharide may include one or more selected from trehalose, sucrose, sucrose ester, maltose and lactose. Examples of polysaccharide may include dextrin. Examples of sugar alcohol may include one or more selected from mannitol, isomaltulose (trade name Palatinose (registered trademark)) and a reduced product of isomaltulose.

The compounding ratio between PRENCSO and saccharide in the dry PRENCSO composition is not particularly limited, and preferably 5 parts by mass or more, more preferably 10 parts by mass or more, more preferably 50 parts by mass or more, more preferably 100 parts by mass or more of saccharide is comprised relative to 1 part by mass of PRENCSO. The upper limit of the compounding ratio is not particularly limited, and typically, preferably 1000 parts by mass or less, more preferably 500 parts by mass or less, more preferably 200 parts by mass or less of saccharide is comprised relative to 1 part by mass of PRENCSO. When multiple types of saccharide are compounded, the amount of saccharide in terms of the compounding ratio refers to the total amount of multiple types of saccharide.

The method for producing the dry PRENCSO composition preferably comprises allowing the porous carrier to absorb a solution containing PRENCSO, and drying the resultant. According to this embodiment, other component present in PRENCSO and the solution is carried on the porous carrier. When the dry PRENCSO composition comprises saccharide, the solution may be a solution containing PRENCSO and saccharide. The solution is preferably an aqueous solution, more preferably an acidic aqueous solution further containing an acid such as hydrochloric acid, particularly preferably an acidic aqueous solution having a pH of 3.5 or less.

The drying method is not particularly limited, and lyophilization or hot-air drying may be exemplified.

The lyophilization is typically a method involving preliminarily freezing a sample at - 80°C to -20°C, and then drying it under reduced pressure. The drying temperature may be any appropriate temperature. Examples of the freezing include a method involving immersion in liquid nitrogen, a method involving loading into a freezer at -4°C to -80°C, and a method involving loading, together with dry ice, into an alcohol freezing bath, but not limited thereto. The drying temperature may be higher than the freezing temperature, and is preferably less than 80°C, more preferably less than 65°C, most preferably in the range of 20°C to 50°C.

The hot-air drying may be performed, for example, under a temperature condition of 40°C to 80°C, preferably about 60°C.

Subsequently, preferable embodiments of the dry enzyme composition are described.

As alliinase, alliinase derived from Alliaceae plants, particularly preferably alliinase derived from garlic, may be used. Alliinase may be purified alliinase or roughly purified alliinase. Roughly purified alliinase is extracted from, for example, garlic by a simple method. Examples of a simple method for preparing roughly purified alliinase include a "method involving adding an acid to an extracted solution of hydrated and pulverized garlic, and subjecting the resultant to isoelectric point precipitation of alliinase, followed by recovering and re-dissolving a precipitated product". The source for the extraction is not limited to garlic.

The dry enzyme composition may further comprise a protecting agent of alliinase. Examples of the protecting agent of alliinase include saccharide, particularly suitably include disaccharide. Examples of the disaccharide may include one or more disaccharides selected from sucrose, trehalose, maltose, lactose, or cellobiose. As the protecting agent of alliinase, a salt may further be comprised in addition to saccharide. Examples of the salt include any salt, for example, KCl, MgCl₂, and NaCl, and a monovalent metal salt is suitable and specifically NaCl is suitable. Further, the protecting agent of alliinase may also include pyridoxal phosphate. In other words, the protecting agent of alliinase preferably (i) comprises saccharide, or (ii) comprises saccharide, and a salt and/or pyridoxal phosphate.

As LFS may be LFS derived from plants belonging to the Alliaceae family (preferably onion), produced by a genetic recombination method, and a variant enzyme thereof, for example, LFS described in Domestic Re-publication of PCT International Publication WO02/020808, and Domestic Re-publication of PCT International Publication WO03/074707. As LFS, it is particularly preferable to use LFS prepared by a genetic recombination method described in Domestic Re-publication of PCT International Publication WO02/020808 (WO02/020808). LFS isolated from Alliaceae plants, in particular, an onion may also be used.

LFS may be purified LFS or roughly purified LFS. Roughly purified LFS may be, for example, LFS extracted from onion by a simple method. Examples of a simple method for preparing roughly purified LFS include a "method involving treating a hydrated and pulverized extracted solution of onion with an anion-exchange resin, and then performing adsorption and elution". The source of the extraction is not limited to an onion.

The compounding ratio between alliinase and LFS in the dry enzyme composition is not particularly limited, and, for example, alliinase may be comprised at 0.02 Units to 200 Units, more preferably 0.5 Units to 50 Units per microgram of LFS. LFS herein may be rLFS used in Examples, and has a specific activity of 6.0 × 10⁶ area/µg.

The porous carrier comprised in the dry enzyme composition is preferably a porous carrier being water absorptive, more preferably a porous carrier containing a hydrophilic polymer such as cellulose and being water absorptive, particularly preferably a water absorptive paper. The shape of the porous carrier is not particularly limited, and is preferably a sheet shape.

The method for producing the dry enzyme composition preferably comprises allowing the porous carrier to absorb a solution containing alliinase and LFS, and drying the resultant. The solution containing alliinase and LFS is preferably an aqueous solution, more preferably an aqueous solution further containing the protecting agent of alliinase. The compounding ratio between alliinase and LFS in the solution may be the same as the compounding ratio in the dry PRENCSO composition.

The drying method is not particularly limited, and lyophilization or hot-air drying may be exemplified. Preferable embodiments of lyophilization and hot-air drying are as described with respect to the dry PRENCSO composition.

Subsequently, preferable embodiments of the kit and the method according to the present disclosure are described.

The dry PRENCSO composition and the dry enzyme composition in the method for generating a lachrymatory factor and the lachrymatory factor generating kit according to the present disclosure are preferably integrated. With the dry PRENCSO composition and the dry enzyme composition being integrated in advance, a lachrymatory factor can be generated on the site where the lachrymatory factor is utilized, only by contacting the compositions with water, without any need for mixing both compositions. The lachrymatory factor generating kit according to the present disclosure, comprising the dry PRENCSO composition and the dry enzyme composition and in which the dry PRENCSO composition and the dry enzyme composition are integrated, can be referred to as a "lachrymatory factor generating tool" or "lachrymatory factor generating composition".

In a more preferable embodiment of an aspect where the dry PRENCSO composition and the dry enzyme composition are integrated, the dry PRENCSO composition and the dry enzyme composition are integrated so that water is permeable. In a further preferable embodiment thereof, the dry PRENCSO composition and the dry enzyme composition are integrated by being bound with an adhesive mean such as an adhesive or a water-permeable double-sided tape and thus integrated. It is particularly preferable in one aspect of these embodiments that both the porous carrier carrying PRENCSO and the porous carrier carrying alliinase and LFS have a sheet shape and the dry PRENCSO composition and the dry enzyme composition be stacked to be integrated.

In another aspect of the kit and the method according to the present disclosure, the dry PRENCSO composition and the dry enzyme composition are adjacently located. The kit according to this aspect can generate a lachrymatory factor when water is diffused entirely upon water being added to at least one of the dry PRENCSO composition and the dry enzyme composition.

In still another aspect of the kit and the method according to the present disclosure, a porous carrier carrying PRENCSO and a porous carrier carrying alliinase and LFS are integrated in one carrier, and PRENCSO is carried on one portion of the one carrier and alliinase and LFS are carried on another portion of the one carrier. In the kit according to this aspect, one portion of the one carrier, on which PRENCSO is carried, corresponds to the dry PRENCSO composition, and another portion of the one carrier, on which alliinase and LFS are carried, corresponds to the dry enzyme composition. The kit according to this aspect can generate a lachrymatory factor when water is diffused entirely upon water being added to the one carrier.

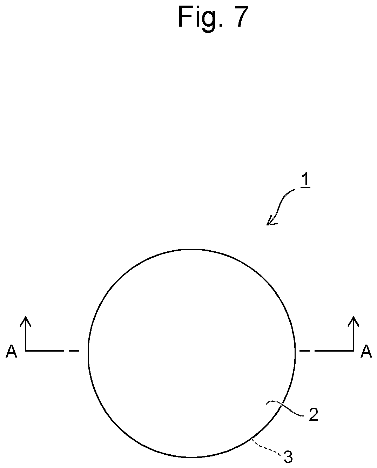

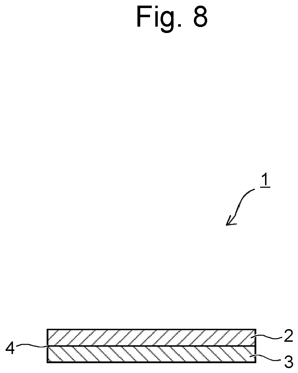

Figure 7 and Figure 8 illustrate one example of a lachrymatory factor generating kit in which the dry PRENCSO composition and the dry enzyme composition are stacked to be integrated. In a lachrymatory factor generating kit 1 in this example, a sheet-shaped dry PRENCSO composition 2 and a sheet-shaped dry enzyme composition 3 are stacked to be integrated. In the lachrymatory factor generating kit 1 in this example, the dry PRENCSO composition 2 and the dry enzyme composition 3 are preferably stacked so that water is permeable in a boundary section 4 between these compositions. Although not illustrated, an adhesive mean such as an adhesive or a water-permeable double-sided tape may be interposed between the dry PRENCSO composition 2 and the dry enzyme composition 3 in the boundary section 4.

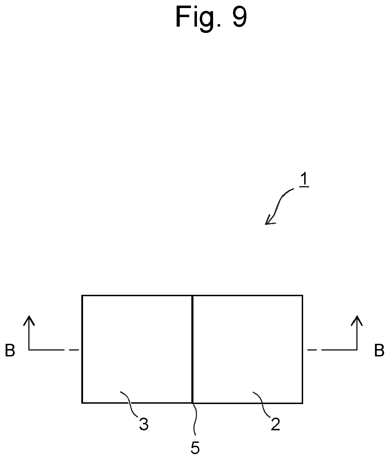

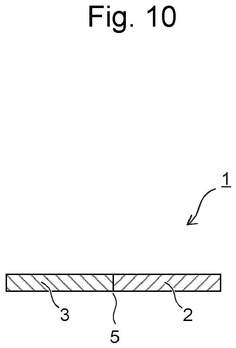

Figure 9 and Figure 10 illustrate one example of a lachrymatory factor generating kit where the dry PRENCSO composition and the dry enzyme composition are adjacently located. In a lachrymatory factor generating kit 1 in this example, a sheet-shaped dry PRENCSO composition 2 and a sheet-shaped dry enzyme composition 3 are adjacently located on one surface. In the lachrymatory factor generating kit 1 in this example, water is preferably permeable in a boundary section 5 between the dry PRENCSO composition 2 and the dry enzyme composition 3. The dry PRENCSO composition 2 and the dry enzyme composition 3 may or may not be bound to each other in the boundary section 5.

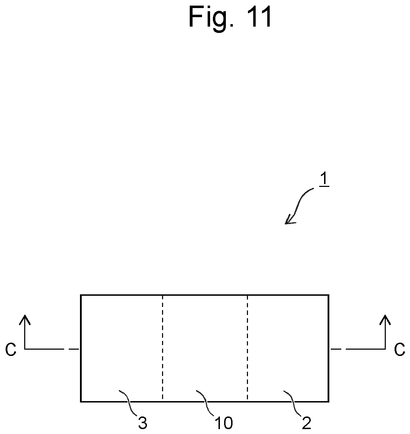

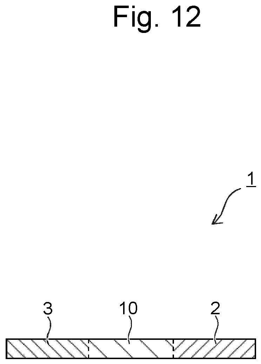

Figure 11 and Figure 12 illustrate one example of a lachrymatory factor generating kit comprising one carrier, in which a porous carrier carrying PRENCSO and a porous carrier carrying alliinase and LFS are integrated, and comprising the dry PRENCSO composition carrying PRENCSO on one portion of the one carrier, and the dry enzyme composition carrying alliinase and LFS on another portion of the one carrier. The lachrymatory factor generating kit 1 in this example comprises the dry PRENCSO composition 2 corresponding to portion where PRENCSO is carried on one portion of a sheet-shaped carrier 10, and the dry enzyme composition 3 where alliinase and LFS are carried on another portion of the sheet-shaped carrier 10.

The water to be contacted with the dry PRENCSO composition and the dry enzyme composition, for generation of a lachrymatory factor, in the method for generating a lachrymatory factor and the lachrymatory factor generating kit according to the present disclosure may be pure water or an aqueous solution containing other component in the water. Examples of such other component include a surfactant. In a case where an aqueous solution of a surfactant is contacted with the dry PRENCSO composition and the dry enzyme composition, a lachrymatory factor tends to be more easily generated compared to the case of using pure water. The surfactant is preferably a non-ionic surfactant or a zwitterionic surfactant, particularly preferably a non-ionic surfactant. Examples of the non-ionic surfactant may include polysorbate 20, polysorbate 60, polysorbate 65, and polysorbate 80. The surfactant is preferably added to the water so as to have a final concentration of 0.005% by mass to 1% by mass. The lachrymatory factor generating kit according to the present disclosure may comprise an aqueous solution containing the surfactant, as a further constituent component.

The subject for the method for generating a lachrymatory factor and the lachrymatory factor generating kit according to the present disclosure is not limited to humans. Specifically, the method may also be applied to mammals such as a monkey, a dog, a cat, a horse, a cow, a sheep, a rabbit, and a mouse.

The method for exposing a LF (lachrymatory factor) to the eye is not particularly limited, and is most efficiently to place the lachrymatory factor generating kit in a lachrymatory cup and to fill the cup with LF, and then the one eye of the subject is closely attached so that the eye is covered with an opening. In this regard, LF having volatility has only to be applied in the eye, and thus tears are also induced by setting the lachrymatory factor generating kit under the eye. The lachrymatory factor generating kit may be set under the eye by directly attaching the lachrymatory factor generating kit under the eye or by using a tool for installing the lachrymatory factor generating kit under the eye, such as goggles.

### Examples

### <Experiment 1>

### 1. Method for preparing PRENCSO pad

### 1.1. Materials

20 mg/ml PRENCSO solution
0.1 N hydrochloric acid
saccharide (lactose)
Thin circular paper filter (ϕ8 mm, thickness 0.7 mm)
-80°C Freezer
Lyophilizer

### 1.2. Method

(1) A 0.67 mg/ml PRENCSO solution in hydrochloric acid was prepared by diluting a 20 mg/ml PRENCSO solution with a mixed solution, in that ultrapure water: 0.1 N hydrochloric acid (15:1, v/v), so that the final concentration of PRENCSO was 0.67 mg/ml.
(2) A 1 mg/ml PRENCSO solution in hydrochloric acid was prepared by diluting a 20 mg/ml PRENCSO solution with a mixed solution, in that ultrapure water: 0.1 N hydrochloric acid (15:1, v/v), so that the final concentration of PRENCSO was 1 mg/ml.
(3) A 1 mg/ml PRENCSO-lactose solution in hydrochloric acid was prepared by diluting a 20 mg/ml PRENCSO solution and lactose with a mixed solution, in that ultrapure water: 0.1 N hydrochloric acid (15:1, v/v), so that the final concentration of PRENCSO was 1 mg/ml and the final concentration of lactose was 100 mg/ml.
(4) Each solution was applied by 30 µl to a circular paper filter, and frozen at -80°C.
(5) After freezing, a PRENCSO pad was obtained by drying with a lyophilizer for 14 hours or more.

### 2. Method for preparing enzyme pad

### (1) Preparation of LFS

A lachrymatory factor synthase (LFS) was produced by a genetic recombination method, according to the method described in WO02/020808.

The outline was as follows. A plasmid (pGEX-4T-3-E2-3-1) expressing LFS protein was introduced to E.coli, by a competent method, in order to obtain a transformant. The transformant obtained was subjected to shaking culture at 37°C in an LB medium containing 100 µg/ml of ampicillin. When isopropyl-β-thiogalactopyranoside (IPTG) was added for the induced production to the medium, a fusion protein of glutathione S transferase (GST) and E2-3-1 (hereinafter, referred to as "GST-E2-3") was accumulated in the body of the bacteria.

The transformant was cultured as described above and the body of the bacteria was collected by centrifugation, and then ultrasonically crushed. The supernatant recovered by centrifugation was allowed to flow in a glutathione sepharose 4 Fast Flow column (manufactured by Amersham Pharmacia Biotech, Inc.), and a GST fusion protein was adsorbed to the column. After the column was washed, the fusion protein was eluted with an elution buffer containing reduced glutathione, and a purified E2-3 fusion protein sample was obtained.

The fusion protein sample was allowed to flow in a HiTrap Desalting column (manufactured by Amersham Pharmacia Biotech, Inc.), to remove the reduced glutathione, and adsorbed to the glutathione sepharose 4 Fast Flow column again. After the column was washed, the column was filled with a buffer containing thrombin, and subjected to protease treatment at room temperature for 2 hours, to cleave a GST tag from the fusion protein. Recombinant E2-3 from which the GST tag was removed was eluted from the column, and then Benzamidine Sepharose 6 was added to the eluate and mixed, and centrifuged to remove thrombin in the eluate, and thus a recombinant E2-3 sample was obtained (which is referred to as rLFS).

### (2) Preparation of alliinase

### (2-1) Pulverization/acid precipitation of garlic

First, a pot of mixer was placed in a refrigerator to cool. A rotor was installed in a low-temperature centrifuge, and cooled at a temperature set to 4°C. An equivalent of a buffer A was added to a garlic piece (100 g), and the piece was pulverized by the mixer. A duplicate gauze on an opening of the pot placed on ice was fastened with an elastic band, and the pulverized product was allowed to flow onto the gauze to filter. After a certain amount of the filtrate was obtained, the pulverized product on the gauze was enclosed with the gauze, and squeezed. The filtrate obtained was centrifuged at 4°C and at 12000 rpm for 10 minutes, and a centrifuge supernatant was recovered. While the centrifuge supernatant recovered was stirred with being placed on ice, acetic acid was added with the pH being monitored, and a pH of 4.0 is achieved. After a pH of 4.0 was achieved, the resultant was left for 5 minutes, as it was. A sample with a precipitate emerged was centrifuged at 4°C and at 12000 rpm for 10 minutes, and a pellet centrifuged was recovered (with the buffer A).

The pellet recovered was adjusted to be at a volume of 50 ml to 100 ml (buffer A), and was left at 5°C for 30 minutes, as it was. The sample was centrifuged at 4°C and at 12000 rpm for 10 minutes. The centrifuge supernatant recovered was prepared with an aqueous 1 N sodium hydroxide solution so as to have a pH of 6.5. This is referred to as a rough extraction solution of alliinase.

### (2-2) Treatment with hydroxyapatite column

The rough extraction solution of alliinase (centrifuge supernatant) was applied to a hydroxyapatite column equilibrated with the buffer A. The solution was adsorbed in the form of a yellow band, to the hydroxyapatite column. The hydroxyapatite column to which such a sample was applied was washed with 300 ml of the buffer A. 300 ml of a buffer C was allowed to flow in the washed hydroxyapatite column for elution. The eluate was fractionated by 10 ml with a fraction collector, and a fraction in which a yellow eluate was fractionated was collected.

### (2-3) Purification of alliinase with ConA column

A 20 mM calcium chloride/magnesium chloride solution in an amount of 1/20 of the amount of the fraction collected (20 ml) was added to increase the concentrations of calcium and magnesium ions in a sample solution. Re-generation was made thereafter, and the resultant was applied to a ConA column equilibrated with a starting buffer (a recommended buffer described in the manual of ConA Sepharose 4B). The ConA column after application of the sample was washed with 50 ml of the starting buffer. 50 ml of a ConA elution buffer (a recommended buffer described in the manual of ConA Sepharose 4B) was allowed to flow in the washed ConA column for elution. The eluate was fractionated by 2 ml with a fraction collector, and a fraction in which a yellow eluate was fractionated was collected.

### (2-4) Method for concentrating alliinase purified

10 ml of the yellow eluate obtained by ConA column purification (alliinase solution) was introduced in CENTRIPLUS CONCENTRATORS (up to 15 ml, No. 4421) (manufactured by Merck Millipore). CENTRIPLUS CONCENTRATORS was installed in a centrifuge cooled to 4°C, and centrifuged at 3000 rpm for 30 minutes, the content was confirmed once, and then centrifuged once again at 3000 rpm for 30 minutes. The activity of the alliinase solution with concentration completed was measured according to the following method, and confirmed to have achieved the required concentration.

The buffer A (pH 7.0) (50 mM buffer A for alliinase purification) was prepared as follows. A 50 mM dipotassium hydrogen phosphate solution (5.22 g dissolved in 600 ml) and a 50 mM potassium dihydrogen phosphate solution (3.4 g dissolved in 500 ml) were prepared. Both were mixed with the pH being monitored, and the pH was adjusted to 7.0. To 9 volumes of the resulted buffer, 1 volume of glycerol was added and mixed well. To 1 L of the resulted glycerol-containing buffer, 5.3 mg of pyridoxal phosphate was added and mixed. The resulted buffer was labelled, and stored in a low-temperature experiment depot (10°C).

The buffer C (pH 7.0) (500 mM buffer C for alliinase purification) was prepared as follows. A 500 mM dipotassium hydrogen phosphate solution (43.6 g dissolved in 500 ml) and a 500 mM potassium dihydrogen phosphate solution (34.0 g dissolved in 500 ml) were prepared. Both were mixed with the pH being monitored, and the pH was adjusted to 7.0. To 9 volumes of the resulted buffer, 1 volume of glycerol was added and mixed well. To 1 L of the resulted glycerol-containing buffer, 5.3 mg of pyridoxal phosphate was added and mixed. The resulted buffer was labelled, and stored in a low-temperature experiment depot (10°C).

### (2-5) Method for measuring activity of alliinase

The activity of alliinase was calculated by a method for measuring the amount of pyruvic acid produced in decomposition of PRENCSO by alliinase.

A 100 mM phosphate buffer (pH 6.5) was used to prepare each of a 0.16 mg/ml pyridoxal phosphate solution, a 3.8 mg/ml NADH solution, a 6.0 µg/ml lactase dehydrogenase (LDH) solution, and 20 mg/ml PRENCSO solution. Using the above pyridoxal phosphate solution, an alliinase solution was diluted 100-fold to prepare a sample. 100 µl of a 0.16 mg/ml pyridoxal phosphate solution, 100 µl of a 3.8 mg/ml NADH solution, 100 µl of a 6.0 µg/ml LDH solution, and 100 µl of a 20 mg/ml PRENCSO solution were added to 2500 µl of 100 mM phosphate buffer (pH 6.5), and these were mixed. To this mixed solution, 100 µl of an alliinase solution, as a specimen, was added to initiate the enzyme reaction, and the change in absorbance of the reaction solution over time was measured for 3 minutes immediately after the start of the reaction. The decreasing rate in absorbance was calculated from the obtained result of the change in absorbance to confirm the decreasing rate in absorbance falling within the range of 0.02 to 0.19. If the decreasing rate in absorbance did not fall within this range, re-measurement was performed with the rate of dilution of the alliinase solution being changed. The alliinase activity (Unit) was calculated from the obtained decreasing rate in absorbance, namely, the amount of change in absorbance per minute.

### (3) Preparation of enzyme pad

To 120 µl of a 0.02 mg/ml rLFS solution, 120 µl of a 50 U/ml alliinase solution (10% trehalose, 1% NaCl, 25 µM pyridoxal phosphate) was added and mixed. This mixed solution was absorbed by 30 µl by a thin circular paper filter (ϕ8 mm, thickness 0.7 mm), and then preliminarily frozen at -80°C under atmospheric pressure. After freezing, lyophilization was performed at -10°C under reduced pressure to obtain an enzyme pad.

### 3. Method for preparing lachrymatory factor generating kit

The PRENCSO pad and the enzyme pad each produced were bonded with a tape paste (DOTLINER from KOKUYO Co., Ltd.) to integrate, and thus the integrated lachrymatory factor generating kit was obtained.

### 4. Comparison test of amount of LF generated (lachrymatory factor)

### 4.1. Materials

Integrated lachrymatory factor generating kit (comprising PRENCSO pad prepared by impregnation with 0.67 mg/ml PRENCSO solution in hydrochloric acid)
Conventional lachrymatory factor generating kit (kit comprising enzyme pad and 0.67 mg/ml PRENCSO solution in hydrochloric acid)
1.5-ml Tube
4-ml Vial bottle with septum
Glass syringe (with needle)
Vortex mixer
0.45-µm Filter
1-ml Syringe

### 4.2. Apparatus

Waters Alliance HPLC

### 4.3. High-performance liquid chromatography (HPLC) analysis conditions

Column ODS (Senshu Scientific Co., Ltd.)
Detection 254 nm
Oven temperature 35°C
Amount of injection 20 µl
Flow rate 0.6 ml/min
Mobile phase (MeOH: TFA water (pH 3.3) = 30%:70%, isocratic)
Sample cooler 5°C

### 4.4. Method

### (1) Generation of LF by conventional lachrymatory factor generating kit

(1-1) An enzyme pad not bonded with any PRENCSO pad, as a conventional lachrymatory factor generating kit described in JP5383075B, was placed in a 4-ml vial, and 30 µl of a 0.67 mg/ml PRENCSO solution in hydrochloric acid was added and the vial was tightly stoppered.

(1-2) After 30 seconds to 3 minutes from the addition of the solution, 500 µl of methanol was added into the vial by the glass syringe, and the resultant was mixed by the vortex mixer for 30 seconds to stop the reaction.

(1-3) 200 µl of the supernatant was filtered, and transferred to a HPLC vial.

(1-4) The peak area of LF at a detection wavelength of 254 nm and a retention time of about 9.9 minutes was determined with HPLC.

### (2) Generation of LF by integrated lachrymatory factor generating kit

(2-1) An integrated lachrymatory factor generating kit, in which a PRENCSO pad prepared by impregnation with a 0.67 mg/ml PRENCSO solution in hydrochloric acid, and an enzyme pad were bonded, was used.

(2-2) The integrated lachrymatory factor generating kit was placed in a 4-ml vial, 60 to 100 µl of water was added from the side of the PRENCSO pad or the enzyme pad, and the vial was tightly stoppered.

(2-3) After 1 minute and 2 minutes from water addition, 500 µl of methanol was added into the vial by the glass syringe, and the resultant was mixed by the vortex mixer for 30 seconds to stop the reaction.

(2-4) 200 µl of the supernatant was filtered, and transferred to a HPLC vial.

(2-5) The peak area of LF at a detection wavelength of 254 nm and a retention time of about 9.9 minutes was determined with HPLC.

### 5. Surfactant addition test

### 5.1. Material

0.05% Tween 20/water

### 5.2. Method

(1) An integrated lachrymatory factor generating kit, in which a PRENCSO pad prepared by impregnation with a 0.67 mg/ml PRENCSO solution in hydrochloric acid, and an enzyme pad were bonded, was used.
(2) The integrated lachrymatory factor generating kit was placed in a 4-ml vial, 80 µl of water or 0.05% Tween 20/water was added from the side of the PRENCSO pad or the enzyme pad, and the vial was tightly stoppered.
(3) After 1 minute from addition of water or 0.05% Tween 20/water, 500 µl of methanol was added into the vial by the glass syringe, and the resultant was mixed by the vortex mixer for 30 seconds to stop the reaction.
(4) 200 µl of the supernatant was filtered, and transferred to a HPLC vial.
(5) The peak area of LF at a detection wavelength of 254 nm and a retention time of about 9.9 minutes was determined with HPLC.

### 6. Pain comparison test

### 6.1. Materials

Integrated lachrymatory factor generating kit 1 (comprising PRENCSO pad prepared by impregnation with 1 mg/ml PRENCSO solution in hydrochloric acid)
Integrated lachrymatory factor generating kit 2 (comprising PRENCSO pad prepared by impregnation with 1 mg/ml PRENCSO/lactose solution in hydrochloric acid)
Conventional lachrymatory factor generating kit (kit comprising enzyme pad and 1 mg/ml PRENCSO solution in hydrochloric acid)
Lachrymatory cup (bottomed container with opening having shape capable of covering one eye)
Double-sided tape

### 6.2. Subject

Males and females in their 30s, 40s, and 60s

### 6.3. Method

(1) The conventional lachrymatory factor generating kit was allowed to generate a lachrymatory factor (LF), by bonding one enzyme pad to the bottom in the lachrymatory cup with the double-sided tape, and dropping 30 µl of a 1 mg/ml PRENCSO solution in hydrochloric acid.
(2) The integrated lachrymatory factor generating kit 1 or 2 was allowed to generate LF, by bonding one kit to the bottom in the lachrymatory cup, with the double-sided tape, so that the PRENCSO pad and the bottom surface were in contact with each other, and dropping 80 µl of water from the side of the enzyme pad.
(3) The opening of the lachrymatory cup was lidded and tightly sealed, and the lachrymatory cup was filled with LF for 1 minute.
(4) Subsequently, the lachrymatory cup from which the lid was removed was closely attached to one eye of the subject so that the eye was covered with the opening, thereby exposing LF to the eye for 120 seconds. The strength of pain felt after the start of exposure was evaluated by the subjects themselves over time, based on the following five-level rating points. The test was carried out for five persons in total (subjects A to E), and the average value was calculated.
(5) After exposure for 120 seconds, tears in the lachrymatory cup were collected, and the amount thereof was measured.

**[Table 1]**

| Rating points of pain strength | |
|---|---|
| Rating points | |
| 0 | Feeling nothing |
| 2.5 | Feeling irritation |
| 5 | Feeling pain |
| 7.5 | Feeling pain, but can open the eye |
| 10 | Feeling pain and cannot open the eye |

### 7. Results

### 7.1. Comparison test of amount of generated LF (lachrymatory factor)

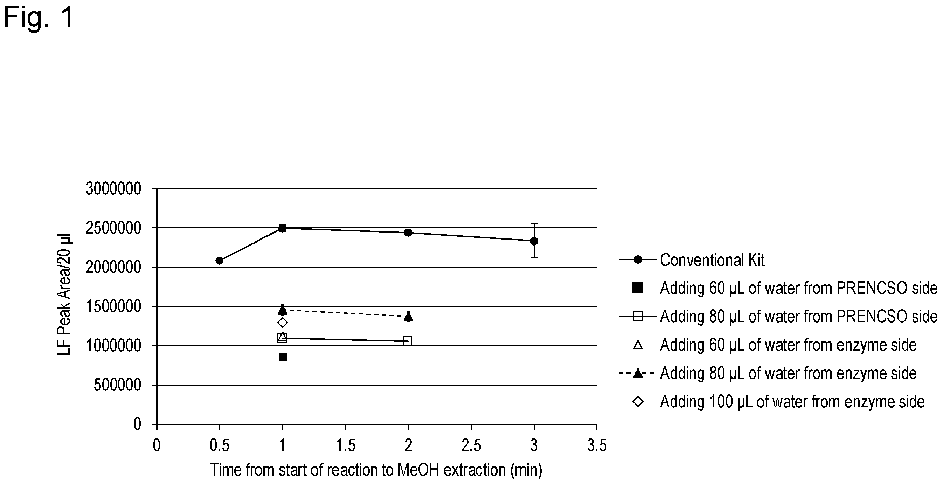

The results of the comparison test of the amount of generated LF (lachrymatory factor) are shown in Figure 1.

The amount of generated LF in the case of dropping water to the integrated lachrymatory factor generating kit, in which the PRENCSO pad and the enzyme pad were bonded, was within the range of 30 to 58% relative to the amount of generated LF in the case of dropping the PRENCSO solution to the enzyme pad of the conventional lachrymatory factor generating kit. It was confirmed that LF was relatively mildly generated in the integrated lachrymatory factor generating kit.

### 7.2. Surfactant addition test

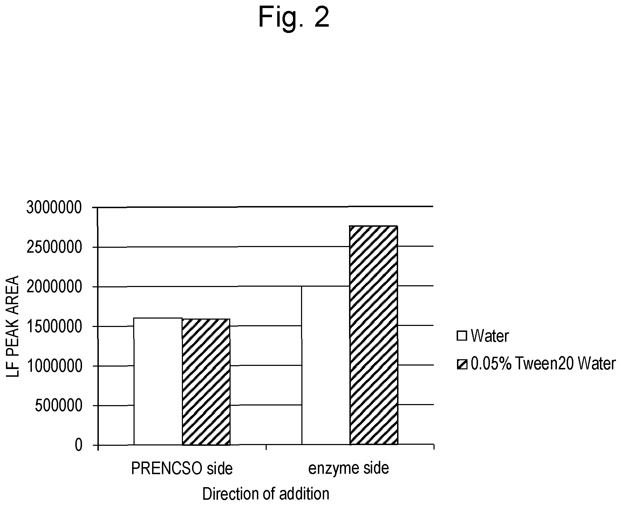

The results of the surfactant addition test are illustrated in Figure 2.

The amount of generated LF when water or 0.05% Tween 20/water was dropped to the integrated lachrymatory factor generating kit from the side of the PRENCSO pad was not confirmed to be different due to whether Tween20 was added.

On the other hand, the amount of generated LF when 0.05% Tween 20/water was dropped to the integrated lachrymatory factor generating kit from the side of the PRENCSO pad was remarkably large compared to that in the case of adding water from the same side.

The foregoing results indicate that generation of LF is promoted by adding a surfactant to water, which is dropped to the integrated lachrymatory factor generating kit.

### 7.3. Pain comparison test

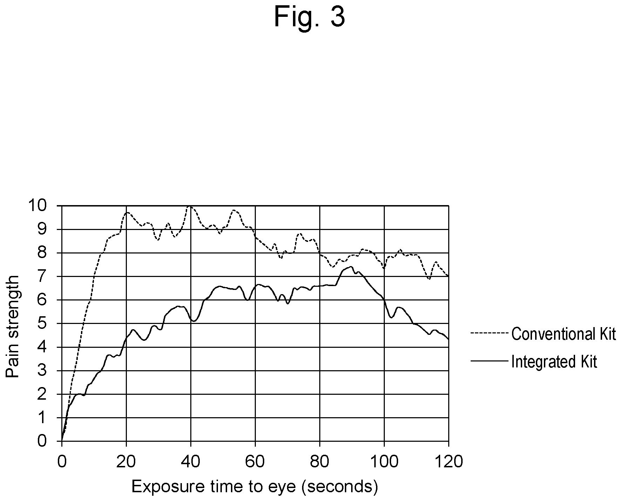

The evaluation results of pain strength in the pain comparison test are illustrated in Figure 3. The graph in Figure 3 illustrates the change in pain strength felt by five subjects in total over time, in which the horizontal axis represents the exposure time to the eye (seconds) and the vertical axis represents the pain strength at each point of time (average value of rating points with a total of 5 subjects), when the integrated lachrymatory factor generating kit 1 (PRENCSO pad containing no lactose) was used (integrated kit) and when the conventional lachrymatory factor generating kit was used (conventional kit), for generating LF.

When the integrated lachrymatory factor generating kit 1 (PRENCSO pad containing no lactose) was used for generating LF, the pain strength was at the maximum value of 7.4, at the average for 120 seconds of 5.3, and at the average after 20 seconds to 120 seconds from the start of exposure of 5.8in. Although not illustrated, the pain strength when the integrated lachrymatory factor generating kit 2 (PRENCSO pad containing lactose) was used for generating LF was comparable with that in the case of using the integrated lachrymatory factor generating kit 1. On the other hand, the pain strength when using the conventional lachrymatory factor generating kit remained no lower than 6.9 after 20 seconds after the start of exposure, and the average for 120 seconds was 8.0, and the average after 20 seconds to 120 seconds from the start of exposure was 8.4.

The amount of tears collected was comparable between the case of using the integrated lachrymatory factor generating kit 1 or 2 and the case of using the conventional lachrymatory factor generating kit.

The foregoing results indicate that the method for collecting tears by exposing generated LF by the integrated lachrymatory factor generating kit 1 or 2 to the eye to induce tears gives less pain and less burden to a subject compared to the method using the conventional lachrymatory factor generating kit.

### <Experiment 2>

1. Integrated lachrymatory factor generating kit
   (1) An integrated lachrymatory factor generating kit 1 was produced by integrating, according to the procedure described in "3. Method for preparing lachrymatory factor generating kit" in Experiment 1, a PRENCSO pad prepared by impregnation with a 1 mg/ml PRENCSO solution in hydrochloric acid according to the procedure described in "1. Method for preparing PRENCSO pad" in Experiment 1 and an enzyme pad prepared according to the procedure described in "2. Method for preparing enzyme pad" in Experiment 1.
   (2) An integrated lachrymatory factor generating kit 2 was produced by integrating, according to the procedure described in "3. Method for preparing lachrymatory factor generating kit" in Experiment 1, a PRENCSO pad prepared by impregnation with a 1 mg/ml PRENCSO/lactose solution in hydrochloric acid according to the procedure described in "1. Method for preparing PRENCSO pad" in Experiment 1 and an enzyme pad according to the procedure described in "2. Method for preparing enzyme pad" in Experiment 1.
2. Storage test
   (1) The integrated lachrymatory factor generating kits 1 and 2 produced were each placed in a 1.5-ml tube and the vial was tightly sealed, and further placed in an aluminum pouch and tightly sealed.
   (2) The resultant was left in a constant-temperature bath at 4°C, 20°C, and 30°C.
   (3) The amounts of generated LF in the integrated lachrymatory factor generating kits 1 and 2 stored for a predetermined period of up to 12 months in the constant-temperature bath at each temperature were determined. The remaining ratio of the amount of generated LF (%) for each of the integrated lachrymatory factor generating kits 1 and 2 was defined as the proportion of the amount of generated LF after storage for the predetermined period at each temperature relative to the amount of generated LF before storage at each temperature, expressed by percentage.

### (4) The amount of generated LF was determined by the following procedure.

One of the integrated lachrymatory factor generating kit 1 or 2 was placed in the 4-ml vial bottle with a septum such that the PRENCSO pad contact with the bottom surface, and 80 µl of water was dropped from the side of the enzyme pad and the vial was tightly stoppered.

1 minute after water being dropped, 500 µl of methanol was added into the vial bottle by the glass syringe, and the resultant was mixed by the vortex mixer for 30 seconds and the reaction was stopped.

200 µl of the supernatant was filtered with a 0.45-µm filter, and the filtrate was transferred to an HPLC vial.

The peak area of LF at a detection wavelength of 254 nm and a retention time of about 9.9 minutes was determined with HPLC. HPLC analysis conditions were as described in "4.3. High-performance liquid chromatography (HPLC) analysis conditions" in "4. Comparison test of amount of generated LF (lachrymatory factor)" in Experiment 1.

### 3. Results

The remaining ratio of the amount of generated LF (%) in each of the integrated lachrymatory factor generating kits 1 and 2 was determined for three samples at each temperature for each storage time, and the average value and the standard deviation were calculated.

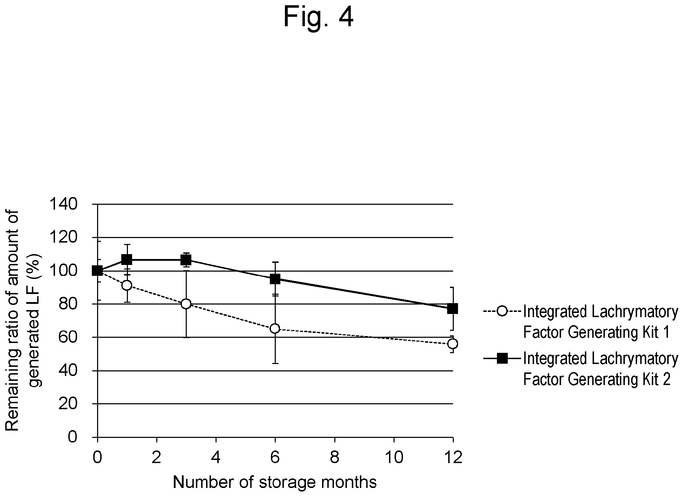

Figure 4 illustrates the average value (error bars representing the standard deviation) of the remaining ratio of the amount of generated LF (%), for each of the integrated lachrymatory factor generating kits 1 and 2, after storage at 4°C for 1 month, 3 months, 6 months, and 12 months.

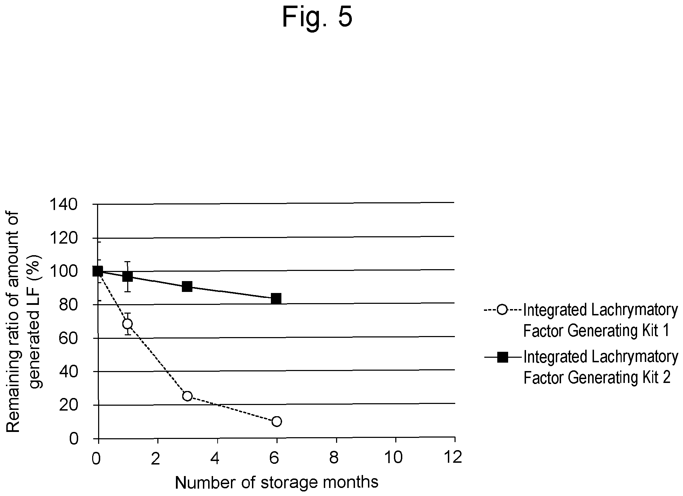

Figure 5 illustrates the average value (error bars representing the standard deviation) of the remaining ratio of the amount of generated LF (%), for each of the integrated lachrymatory factor generating kits 1 and 2, after storage at 20°C for 1 month, 3 months, and 6 months.

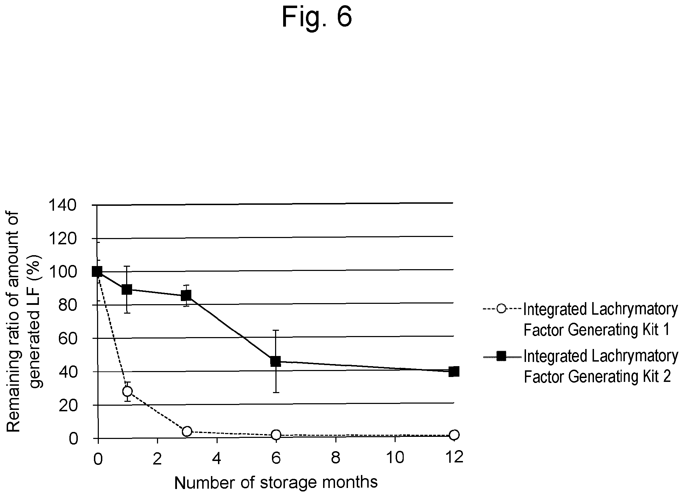

Figure 6 illustrates the average value (error bars representing the standard deviation) of the remaining ratio of the amount of generated LF (%), for each of the integrated lachrymatory factor generating kits 1 and 2, after storage at 30°C for 1 month, 3 months, 6 months, and 12 months.

It was confirmed that the integrated lachrymatory factor generating kit 2 comprising the PRENCSO pad containing lactose was high in remaining ratio of the amount of generated LF (%) after storage at each temperature compared to the integrated lachrymatory factor generating kit 1 comprising PRENCSO pad containing no lactose.

All publications, patents, and patent applications cited herein are herein incorporated by reference as they are.

## Claims

1. A lachrymatory factor generating kit comprising:
(1) a dry PRENCSO composition comprising PRENCSO and a porous carrier carrying it and being dried; and
(2) a dry enzyme composition comprising alliinase, a lachrymatory factor synthase (LFS) and a porous carrier carrying them and being dried.

2. The lachrymatory factor generating kit according to claim 1, wherein (1) and (2) are integrated.

3. The lachrymatory factor generating kit according to claim 1 or 2, wherein (1) further comprises sugar.

4. The lachrymatory factor generating kit according to claim 3, comprising 5 parts by mass or more of sugar relative to 1 part by mass of PRENCSO.

5. A method for generating a lachrymatory factor, comprising
contacting water with
(1) a dry PRENCSO composition comprising PRENCSO and a porous carrier carrying it and being dried, and
(2) a dry enzyme composition comprising alliinase, a lachrymatory factor synthase (LFS) and a porous carrier carrying them and being dried.

6. The method according to claim 5, wherein (1) and (2) are integrated.

7. The method according to claim 5 or 6, wherein (1) further comprises sugar.

8. The method according to claim 7, wherein 5 parts by mass or more of sugar is contained relative to 1 part by mass of PRENCSO.

9. The method according to any one of claims 5 to 8, wherein the water is an aqueous solution containing a surfactant.
